# EUROPEAN PATENT SPECIFICATION

(11) **EP 3 332 708 B1**
(45) Date of publication and mention of the grant of the patent: **31.07.2019**
(21) Application number: 16202878.1
(22) Date of filing: 08.12.2016
(51) Int. Cl.: A61B 6/00, B65B 67/12

(54) **PROTECTIVE BAG DISPENSING SYSTEM FOR A MOBILE X-RAY APPARATUS**
SCHUTZBEUTELAUSGABESYSTEM FÜR MOBILE RÖNTGENVORRICHTUNG
SYSTÈME DE DISTRIBUTION DE SACS DE PROTECTION POUR UN APPAREIL À RAYONS X MOBILE

(43) Date of publication of application: 13.06.2018
(73) Proprietor: AGFA NV, 2640 Mortsel (BE)
(72) Inventor: Elen, Sabina, 2640 Mortsel (BE); Nebosis, Rainer, 2640 Mortsel (BE); Elian, Naser, 2640 Mortsel (BE); Leblans, Paul, 2640 Mortsel (BE)
(74) Representative: Verbrugghe, Anne Marie L.

(56) References cited:
- JP-A- 2011 160 913
- JP-A- 2012 215 699
- JP-A- 2014 204 823
- US-A- 4 537 330
- US-A- 5 123 535
- US-A- 6 126 314
- US-A1- 2004 066 899
- US-A1- 2011 286 575
- US-A1- 2013 064 351
- US-A1- 2014 029 720

## Description

### Technical Field

The present invention relates to a system for bagging a mobile x-ray detector from a mobile x-ray device into a hygiene protective cover. The bagging system can be fitted to a pre-existing mobile X-ray unit and is designed as such that it does not interfere with the handling of the mobile X-ray device by the operator during the movement of the cart.

### Background Art

Mobile x-ray solutions play an important role in diagnostic imaging of patients who lack mobility. Efficient mobile bedside imaging solutions do exist for many years, and are providing patients and staff with the comfort of not having to mobilize or transport the patient for his imaging examination. The usability of mobile X-ray equipment has improved over the past decade thanks to the development of better battery solutions, giving the units sufficient power autonomy for multiple examinations without recharging.

A typical mobile x-ray apparatus comprises a mobile unit on servo driven wheels and a tube arm on which an x-ray source is mounted. In order to ensure enhanced flexibility, a wireless flat panel detector (FPD) is provided which may be slid under the patient directly or is positioned under his bed. In other configurations, the wireless flat panel detector (also called wireless DR-detector) may be replaced by less expensive CR-detectors. In the context of this invention, both types of detectors are interchangeable.

In order to have an improved infection control and in order to protect the sensitive and expensive x-ray detector, the x-ray detector is optionally covered before an x-ray image is made and is positioned under the patient with a disposable hygiene protection means, e.g. a sterile plastic bag of form and size corresponding with the detector. Since a wireless x-ray detector has a relatively high weight due to the integrated battery and electronics (typically 2,5 kg upto 7kg) the operation of covering or bagging such detectors with plastic foils or bags is usually a cumbersome task for the medical staff to perform when preparing the exposures for a new patient. The bagging task typically requires the assistance of a second person who can keep the bag in an opened position, so that the first person can hold the detector with both hands to insert the relatively heavy detector in an easy way.

In order to overcome the need for an assisting person, special adaptations may be provided by the manufacturer of such a mobile x-ray system to make this bagging process easier for a single person to accomplish the task. The dispositions described in the art are often built into the cart of the mobile x-ray device which accommodates the main parts of the x-ray system. As an example of such a disposition, the bagging system described in patent application EP 2878264 A1 refers to adaptations made to the posterior part of the cart, in which the detector storage bin is fitted with a detector holding means to facilitate the bagging operation. The system does however not foresee a space or location for storing the bags themselves.

Alternative solutions provide for similar approaches by complementing the existing detector storage location with an additional slot-like disposition which has then the capability of supporting the detector and at the same time storing the protective bags. Such systems are mostly designed into the cart in the vicinity of the existing detector storage space. The drawback of such systems is that they take up additional space and make the entire system more bulky. The supplementary slot storing the bags takes up additional space in the vicinity of the detector storage area. This additional space may in the end hinder the operator during displacement of the cart; the expanded detector storage space may hinder the knees or lower legs of the operator while walking and moving the cart.

While built-in bagging solutions as referred to above may provide the most aesthetically appealing and design-wise elegant solutions, many of the commercially available mobile x-ray solutions do not provide such supporting option for bagging the detector at all.

Document JP2014204823 discloses a bag dispensing system, according to the preamble of claim 1.

As explained above, the solutions described in the art for bagging the detector are often fully integrated into the cart, which may be preferable from an aesthetic point-of-view.

One of the drawbacks of such integrated detector bagging solution is that the ideal storage conditions for the protective bags are often not respected. Even when used in non-sterile environment, protective bags are recommended but require the necessary attention during storage and handling. For instance, it should be avoided that the protective bags are excessively manipulated or touched, and that they are stored in a hot and/or humid environment as this could induce bacterial growth. Because of the complete integration of the storage cavity for the bags into the body of the cart, the heat dissipating from the different electronic components inside the cart body of the mobile x-ray device unavoidably increases the temperature to which the bags are subjected for the duration of the storage. Depending on the number of bags which can be accommodated in the storage space, this period can extend from a couple of days upto a couple of months. Storage of the plastic bags at a too high temperature and during extended periods deteriorate the mechanical quality of the plastic bags (make the bags more brittle) and could stimulate undesirable bacterial growth and subsequent contamination.

Another problem associated with built-in solutions arises when a bag or a set of bags becomes contaminated by, for instance, an accidental spillage of a liquid into the bag storage area, after which the entire mobile x-ray unit needs to be temporarily decommissioned for a cleaning session.

However, since most of the currently deployed mobile x-ray systems are not foreseen with a supporting system for bagging the detectors, there is a real need for a solution which offers the required functionality, and which can be fitted to existing mobile X-ray systems which lack said functionality. In that respect, multiple contributing problems need to be resolved in the sense that the proposed solution needs to be compatible with the shape of the existing mobile x-ray systems, and should offer sufficient ergonomics in order to improve the overall bagging process, while not undermining the existing comforts of the mobile x-ray unit by itself.

In case that there are no special adaptations foreseen to support the bagging operation, the process will remain cumbersome unless a suitable adaptation can be added (or "retro-fitted") to the mobile x-ray device in question.

### Summary of invention

The present invention provides a system for an improved flat panel detector bagging system which solves a combination of problems found in the art. The system disclosed by this application is a solution for bagging detector systems of mobile x-ray devices which have not been foreseen with this bagging functionality by design. In other words, the present invention solves the problems as referred to above, and in addition to that also provides an important commercial advantage in the sense that the bagging solution is retrofittable to existing mobile x-ray devices. The invention provides for a system as set out in independent claim 1.

The above-described aspects are solved by a system as set out in claim 1.

Specific examples and preferred embodiments are set out in the dependent claims.

In the context of this invention, the protective bags are most frequently plastic bags (PE or PP) which have a similar rectangular shape as the detector cassette the size of which is slightly exceeding the actual size of the detector cassette in order to ensure that no excessive force needs to be exercised on the bags themselves during the bagging operation. Also the bags play a second role to protect a detector from the bodily liquids which may occasionally be present. The bags are most frequently side weld and have a heat sealed edge along the length of each side. The protective bags may be transparent and typically serve only a single usage (one bag per patient). In a preferred embodiment the bags are typically grouped together in a set of 25, 50 or 100 pieces, sharing a sealed portion at the top of the set, under which a line of perforations is located for easy tearing of the bags individually. In some cases the strip at the top of the set above the line of perforations comprises a series of holes at typically equidistant positions to allow to pin up the set of bags onto a bag holding or clamping means. This disposition then comprises a row of hooks or pins at the same (typically equidistant) positions as mentioned above to fit the holes in the strip of welded together bag material above the perforated line. The bag holding means may be implemented in different ways; i.e. as a clamp which keeps the stack of bags together, or by means of a series of hooks as described above, or, as a combination of both. Other bag holding means may be envisaged, such as a roll of bags which is mounted on a spindle or axis, which similarly keeps a multitude of bags individually available for use in the bagging operation.

In the context of this invention, a mobile x-ray apparatus is a highly integrated but complete x-ray device, comprising all essential components to generate and capture digital x-ray images. A mobile x-ray system is capable of operating (for a limited time period) independently from a power grid as it is battery operated. The device is mobile in the sense that it can be moved from room to room requiring only one operator to perform this movement. Most of the key components of the X-ray device are integrated into a trolley or in other words, a cart on wheels. Nevertheless, most of these mobile x-ray devices are still quite bulky and very heavy. Available space in and around the cart is limited, which is a factor taken into account during the design of these systems.

When looking at the orientations of the cart, the rear of the cart has to be understood as the part which is pushed by the operator when moving the cart around. The rear of the cart is the side where the pushing handles are mounted. It is mostly also in this rear area that the detector storage bin is situated, because of the convenience of this location in the workflow of the operator. When the operator positions the cart at -for instance- the bed side of the patient so that the X-ray collimator is positioned straight over the patient, he will be doing the fine positioning using the pushing handles of the cart. Conveniently, the detector can then be picked up easily from the storage bin situated very close to these handles. The positioning of the detector is the next step in the workflow. In principle, the other sides of the cart (left, right, front and top) are also operator accessible, but these areas are less convenient for storing the detector as they are remote from the standing position of the operator during the manoeuvring of the cart. The invention is advantageous in the sense that it can improve the workflow performed by a user on condition that the device is mounted on the preferable location of the cart of the mobile x-ray device.

At the same time, the invention solves the problem of foreseeing adequate support for storing the plastic bags in between bagging operations under optimal conditions. The system foresees sufficient space to store multiple bags or a bundle of bags into the protected space of the cavity formed by the front and back panel. The system also ensures that the plastic bags can be dispensed individually, and that only one bag is being dispensed per bagging procedure. The fact that the bagging system is fitted or mounted at the exterior of the mobile cart, allows that the bags can be protected against the heat dissipation of the equipment inside the cart, but also provides a further technical effect in the sense that the device itself is much better accessible for cleaning. Indirectly, this resolves the issue of a potential undesired downtime period of the entire device in case that the bag storage space needs to be cleaned.

In general the dispensing system shall be placed ideally very close to the storage bin of the panel to allow a very fast workflow for bagging the digital detector. While the bagging system of the invention is an apparatus which is for that reason preferably mounted to the rear side of a mobile cart, there is -as a consequence- an expansion of the overall outside dimensions of the mobile x-ray cart as a whole. More specifically, the rear side of the cart, where the bagging system is preferably mounted, extends further towards the operator.

This expansion however reduces the available legroom for the operator pushing the cart and could lead to an injury of legs, knees or feet during transportation. Especially, since the total length of the dispensing system which hangs downwards from the position where it is mounted to one of the sides of the cart exceeds easily 45cm (a typical large size detector measures 43 x 35 cm), it is clear that the device in its open position causes this physical conflict. Our invention is however advantageous in that it solves this conflict and thus increases the ergonomics of the device by means of a segmented structure.

Another advantageous effect of the invention is that the ergonomics for the operator are further improved during the bagging operation itself; the lower end of the cavity, located between the front and back panel of the device, and are always connected to each other, provides support for the detector when inserted into the cavity. The lower end of the detector touches the bottom of the cavity right after that the plastic bag came off from the bag holding means. The lower end thus serves two purposes towards the user; when the detector touches the bottom of the cavity it is indicated that the detector reached a final point indicating that the bagging is completed. But the bottom of the cavity can also serve as a rest position for the (heavy) FPD, and prevents that the detector drops on the floor. The detector can be temporarily stored in the dispensing system in this position.

The present invention is beneficial in that it allows that any standard mobile x-ray cart can be fitted with a bagging system, while foreseeing the required functionalities needed for adequately bagging a mobile DR-detector cassette. The bagging system also is adapted in order to be fitted to the outside of an existing mobile x-ray cart, while it is fitted with a reclining part so as to not hinder an operator during the manipulation of the cart.

Different features are foreseen in the system to contribute to different particular aspects associated with the bagging process.

Further advantages and embodiments of the present invention will become apparent from the following description and drawings.

### Brief description of drawings

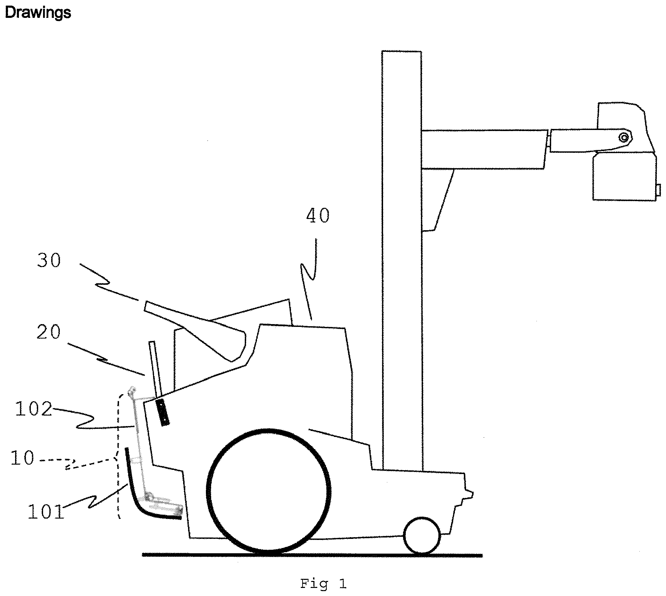

Fig. 1 depicts a mobile x-ray device comprising an embodiment of the invention [10] mounted at the rear of the cart in a closed position. The drawing shows the protective bag dispensing unit [10] mounted at the rear end of the cart [40] of the mobile x-ray device, where it is mounted under the pushing handle [30] and in the vicinity of the mobile x-ray detector [20] stored in the storage bin of the cart. The front panel element [101] and back panel element [102] are shown in a closed position.

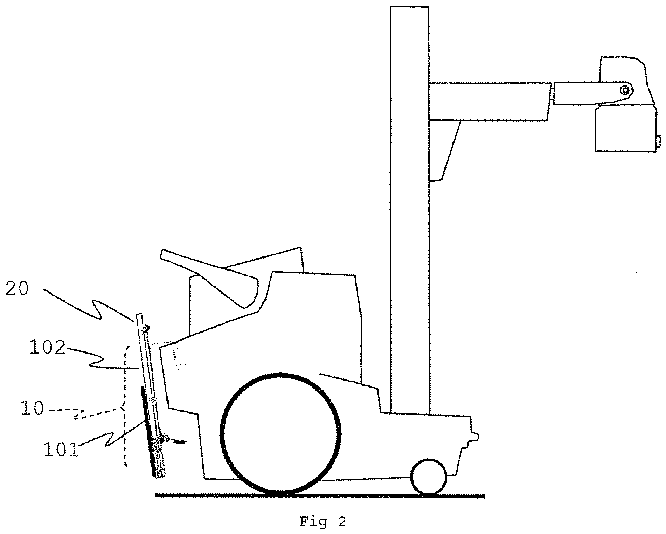

Fig. 2 depicts the same mobile x-ray device comprising an embodiment of the same protecting bag dispensing unit [10] as in Fig. 1, but shown in an open position. The mobile x-ray detector [20] has been transferred from the cart storage position into the protective bag dispensing system [10] which is depicted in an open position. The segments of the back panel element [102] are now aligned as to form a straight surface.

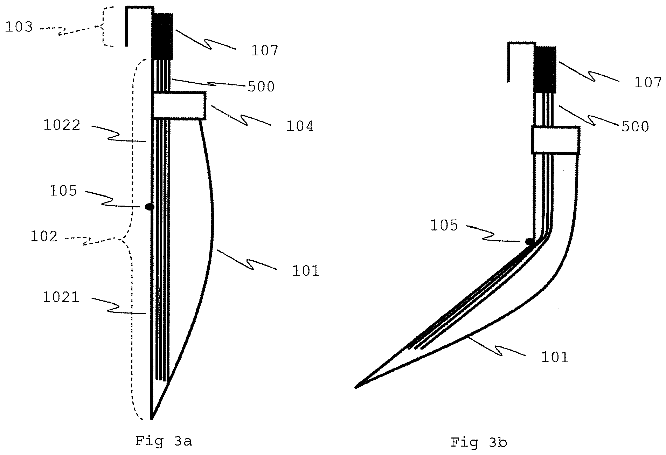

Fig. 3 are two side views of schematic representations of a preferred embodiment of the invention depicted in the "open" (parked or in use)(Fig 3a) and "closed" (transport)(Fig 3b) position. The drawing shows the essential features of the protective bag dispensing system [10]. The drawing depicts the back panel element [102], and shows the invention in an embodiment with 2 segments: the upper segment [1022] and the lower segment [1021]. The upper segment [1022] comprises a fixing means [103] and both segments are connected via a hinge [105]. The front panel element [101] is here depicted as a flexible element which is connected with the back panel [102] at the bottom side of both elements. A lateral spacer element [104] defines a distance between the upper sides of both panels [101] and [102], so as to form a cavity into which a mobile x-ray detector can be introduced. The set of protective bags [500] is fixed at the top side of the back panel element [102] by means of a bag holding means [107]. In fig 3b, the lower segment is hinged backwards (towards the cart wall which is not visible in the drawing) so as to put the dispensing system [10] into its "closed" position. As can be seen in the drawing, the set bags align with the flexed lower segment, and are covered by the front panel element [101].

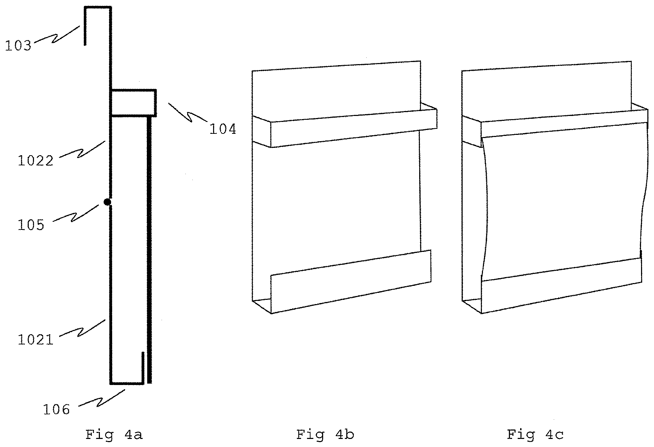

Fig. 4a shows a different embodiment of the dispensing system [10] wherein a rim [106] for supporting a detector cassette is shown, as well as two lateral spacer elements implemented as a rigid strap [104] which allows to introduction of a detector cassette in an aligned way against the back panel element segments [1021] and [1022]. The lateral pieces of rigid strap can be understood as lateral spacer elements, keeping the front panel element [101] separated at a defined distance from the back panel.
Fig 4b shows the embodiment without a complete front panel element, but only a rigid strap [104]. Fig 4c shows the embodiment including a flexible front panel element [101] covering the storage cavity of the dispensing system with a soft and flexible material.

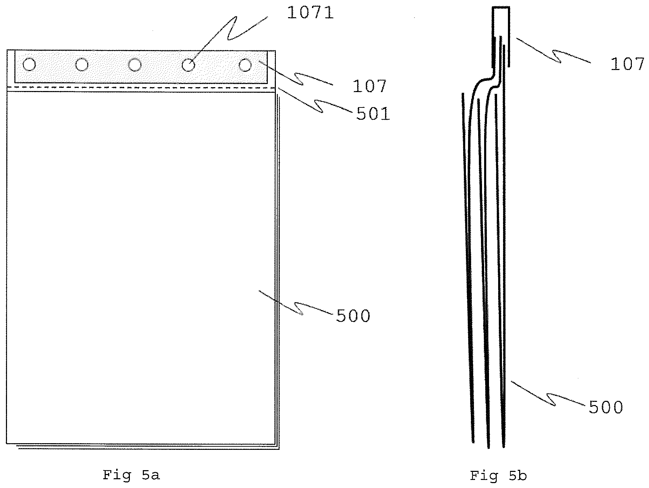

Fig 5a and b represent drawings from respectively a frontal and lateral view of a set of protective bags [500] kept together by a bag holding means [107]. The bag holding means consists of a rigid plate [107] with holes [1071] in it, which can be fixed against the upper side of the back panel element, leaving the openings for a similarly spaced (as the holes) set of pins traversing through the set of bags and keeping them as such together. A line of perforations [501] in each of the bags allows the easy separation of the first available bag from the set of bags.

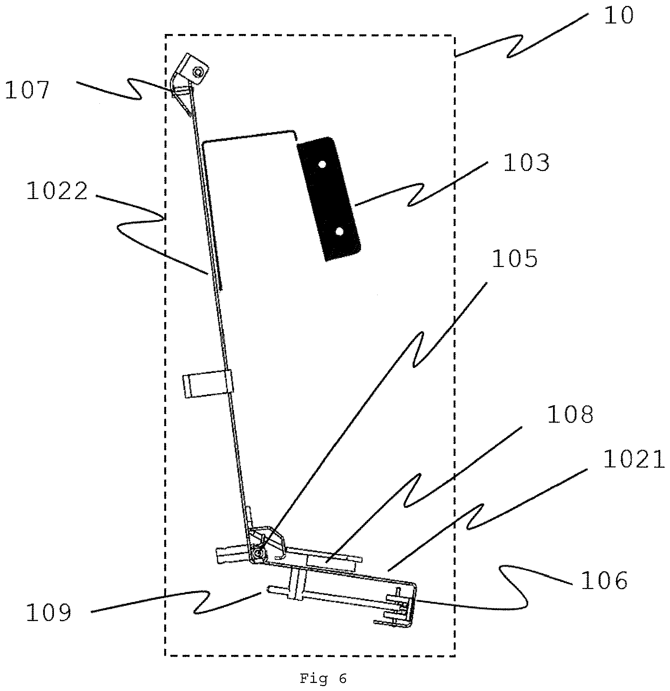

Fig 6 shows an embodiment of the bag dispensing system [10] in its closed position, but in more detail and in a side view. The embodiment shows additional features which may be implemented in the system: [107] represents a clamp which serves as the bag holding means, [103] is an embodiment of a fixing means for the device to the cart of a mobile x-ray system, [1022] the upper segment of the back panel element, and [1021] the lower segment. Also the hinge [105] and the supporting rim [106] are shown. [109] is a joint locking mechanism, while [108] is a fixation means which maintains the lower segment [1021] in a fixed position while the dispensing unit is in a closed position.

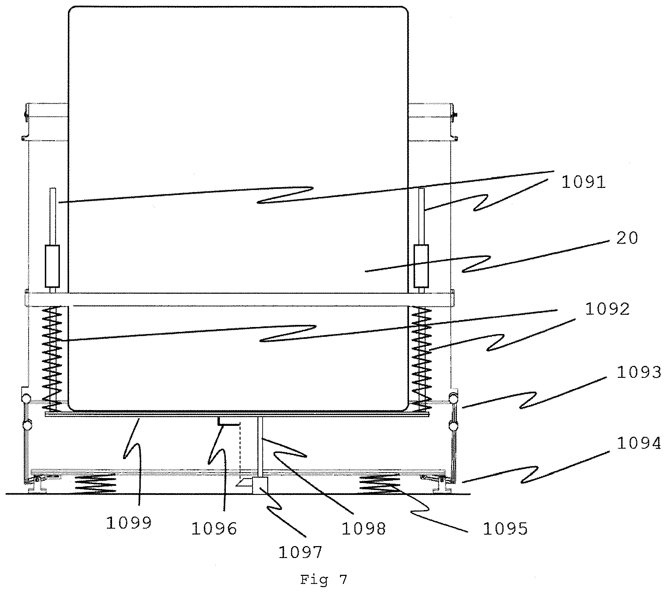

Fig 7 depicts a frontal view of the embodiment of the bag dispensing system [10] with a detailed view of the joint locking mechanism [109]. The dispensing system is here in an open position. The mobile x-ray detector [20] rests on a receptacle [1099] which provides upward pressure to the bottom of the detector by means of the springs [1092]. The guiding shaft [1091] guides the movement of the receptacle [1099]. The guiding shaft [1091], springs [1092] and receptacle [1099] are therefore attached to each other and to the upper segment [1022] of the back panel element [102]. The lift up lock [1097], locking hook [1096], springs [1095], lever [1094] and joint locking [1093] associated with the lower segment [1021] provide the locking and releasing mechanism between the open and closed position of the bag dispensing system.

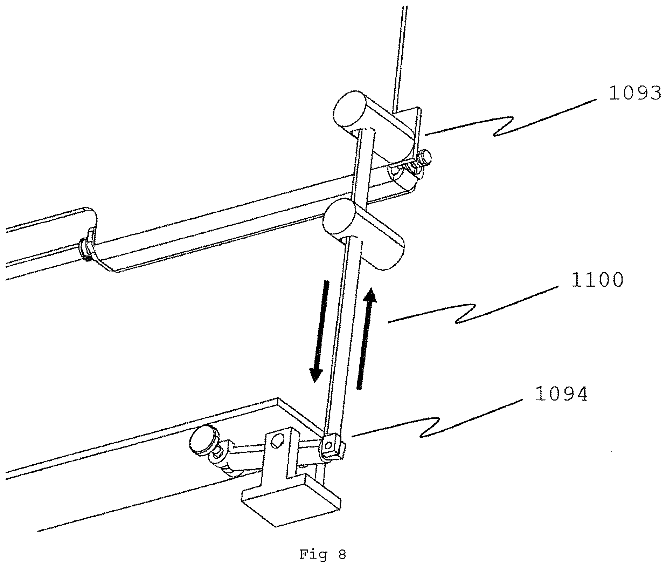

Fig 8 represents a perspective view of the joint locking mechanism, and indicates the movement of the locking latch [1100] (indicated as arrows) into the joint locking [1093] when the lever [1094] is actuated.

### Description of embodiments

In the following detailed description, reference is made in sufficient detail to the above referenced drawings, allowing those skilled in the art to practice the embodiments explained below.

Fig. 1 represents an overview image of a mobile x-ray cart to which an embodiment of our invention [10] is attached. The device is firmly, but removably attached to the cart by means of a fixing means [103] which may be executed in very different ways. The fixing means has the purpose to firmly fix the device to the rear end of the cart while leaving the option open to remove the device for maintenance and cleaning purposes. Therefore, the fixing means could be implemented as a simple set of screws which are screwed through a number of holes in the upper area of the back panel element [102], or could be executed as a more sophisticated clamp mechanism which would be inserted (partially or completely) into the existing detector storage bin of the cart (the storage bin location would be typically also at the rear end of the cart). An example of such a fixing means is depicted in more detail in Fig 6 as [103], where the fixing means is executed as a metal hook which hangs over the edge of the detector storage bin, and of which a portion may be inserted into the storage bin itself. As an example, the black portion [103] in Fig.6 is fixed at the interior of the storage bin, and comprises two holes to fit screws in order to attach the fixing means (by means of said screws) to the interior side walls of the detector storage bin. Another variation is depicted in Fig. 3a and 4a. Many alternatives to achieve the same effect may be conceivable.

The rear area of the cart is the side which is pushed while moving the cart forward. At this location, typically a cart pushing handle [30] is located to allow easy pushing of the heavy device. It is also at this side that the detector storage bin is located (in Fig 1, the detector [20] is partly visible, and is partially inserted into the detector storage bin). This detector storage bin provides a safe storage location for the detector [20] while moving the mobile x-ray device around. It is good practice for the operator to use this storage bin for storing the FPD detector during transportation to avoid damage. The detector storage bin is ideally located close to the cart pushing handle [30], because as soon as the mobile cart is positioned close to the patient in preparation of an exposure, the operator will have his hands on the pushing handle which is conveniently close to the detector storage bin.

In order to capture a diagnostic image, the FPD needs to be positioned manually by the operator every time an exposure is prepared. Quick access to the location where the detector is stored is therefore convenient. The detector should be easily accessible by the operator as soon as the mobile x-ray device is moved into position to prepare for the exposure.

It is for that reason that the embodiment shown in Fig. 1 also locates the protective bag dispensing system [10] as close as possible to the detector storage bin and pushing handle [30], where it is intended to be fixed to the mobile cart with the fixing means [103] as explained before. Nevertheless, other attachment or fixation locations for the protective dispensing system may be envisaged.

In a preferred embodiment of this invention, the bag dispensing system comprises a back panel element [102] and a front panel element [101]. The back panel element is made of a rigid material so that when it is fixed to the cart, it maintains its rectangular shape by its own stiffness and is capable of supporting other elements of the bag dispensing system, but also the detector during the bagging operation. In the preferred embodiment as shown in Fig 3a and 3b, the back panel element is split in two segments [1021] and [1022], named the lower and upper segment respectively. When segmented, both segments can be of the same or different rigid materials. Segmentation into more than two segments can be envisaged as well in order to gain more possibilities for flexing the rigid back panel at different levels. The purpose of the segmentation of the back panel element [102] into multiple segments is to adapt the shape of the back-side of the bag dispensing system to the contours of the cart of the mobile x-ray device to which it is attached, when in a closed position. The effect of this shape adoption is to save valuable legroom below the bag dispensing system, preventing the operator to injure himself while pushing the cart by kicking against the extended back panel element when in its open position. This shape adoption can minimally be achieved using 2 segments, where the lower segment is flexed backwards against the cart wall. But it can be envisaged that a separation of the pack panel into more than two segments can contribute to a better adoption of the shape. Another possibility would be to adapt the shape of the lower segment to the shape of the portion of the cart wall against which it can be flexed against when in said closed position.

In a preferred embodiment, the multiple segments of the back panel element [102] are connected to each other with hinges [105] (e.g. so-called piano hinges) as to form a flexible surface which has limited degrees of freedom (the segmented surface can only bend around the axes of the hinges). As a further elaboration, an embodiment, which is not part of the invention, having an unsegmented but flexible back panel element could be envisaged which even more optimally would be able to adopt the shape of the cart body. Special measures should in that case be taken as to force or guide the flexible back panel element backwards against the cart surface. This could be achieved by tensing the back panel element by applying tensors, springs or tightening dispositions.

As already mentioned, the bag dispensing system knows two operating positions, named the "open" and "closed" position. In the open position, the system has the back panel element [102] in its linearly extended state, allowing the full length of an inserted detector cassette to align to the back panel element. In this position, the detector can be easily inserted into the bag dispensing system, in the cavity made up by the front [101] and back panel element [102]. (See Fig 3a). In the closed position, at least one segment (one or more of the lower segment(s)) of the back panel element is flexed backwards in the direction of the cart wall against which the device is mounted. Since flexing this lower segment [1021] against the cart wall requires that some force has to be applied in the direction of the cart wall (at least when this position has to be maintained during the manipulations of the cart), a mechanical solution to deliver this force has to be applied. In one embodiment, this force is delivered by a spring in the hinge capable of flipping the lower segment backward. In another embodiment, a magnet system [108] has been foreseen, keeping the lower segment retracted by means of magnetic attraction of a metal portion of the lower segment, or a disposition attached to it. This solution is depicted in Fig 6.

Alternatively, more complex configurations (Fig 7) can be envisaged which do not only push the lower segment backwards against the outer wall of the cart against which the bag dispensing system is attached, but which also lock the lower segment [1021] into its desired (straight) position when the bag dispensing system is in its open position. In Fig 7, an example of such a more functional and complex mechanism is disclosed; the mechanism guides an inserted detector cassette onto a receptacle [1099] on which it applies a downward force. Said downward force (Fig 8) actuates a lever [1094] which reverts the downward force and pushes a locking latch [1100] into a locking mechanism [1093], which in that position prevents the lower segment of the back panel element to hinge away from the straight aligned configuration of the dispensing systems' open position. The downward position of the receptacle [1099] is contravened by the springs [1092] which push said receptacle back up, guided by the guiding shafts [1091]. However, when sufficient force is applied on the detector to push the locking hook [1096] (attached to the receptacle) downwards beyond the position of the lift up lock [1097], the receptacle [1099] and the locking mechanism will remain in said locked position until the lift up lock [1097] is released. This release takes place when the detector is again pushed in a downward direction to apply force on the lift up release [1098] which unlocks the lift up lock [1097].

Alternative solutions to the problem to resolve the potential conflict between the lower side of the panels when in an open position and the lower legs or knees of the operator could be to either provide a flexible front panel, or a combination of a flexible front and back panel. Flexible front panels (for instance made of some sort of cloth or flexible plastic) have the disadvantage of being less strong and durable. As explained above, a combination of a flexible front and back panel element would however require a different flexing mechanism which pushes or pulls the joint edge towards the wall of the cart. In this scenario, there is also a risk that the entire bagging system could become clogged up in certain moving parts of the mobile x-ray device, such as the wheels or the x-ray tube arm. It is therefore an object of our invention to foresee a rigid, or at least segmented rigid back panel element, resolving the problems above.

Important aspects for a protective bag dispensing system are to provide a mechanism and space to foresee a sufficient supply of bags while offering the functionality to conveniently dispense individual bags during the bagging operation. The storage space should provide the optimal storage conditions for storing the protective bags with the intent to minimize the risk of material deterioration and undesired development or growth of germs.

Fig. 5a shows an embodiment of a bag holding means [107] to hold a bundle of bags [500] together at their top side. The set of bags comprises a line of perforations [501] which is present in each individual bag of the pile. The line of perforations is foreseen parallel to the top edge of each bag. The lower side of the bag holding means [107] presses against the pile of bags at the top end, but leaves the line of perforations uncovered by a few millimetres, allowing an easy removal of every bag individually.

When a detector cassette is inserted into a protective bag, it needs to be opened first. In other words, one side of the bag needs to be separated from the other side to bring it in an open position. For that, only the first perforated line of the bag may be broken or teared; the second perforated line needs to remain in intact in order for the bag to remain in the right position for inserting the detector in it. When the set of bags have perforated lines on both sides of the bag, there is a risk that an operator tears off the entire bag, by tearing of breaking both perforated lines simultaneously. In order to prevent this, it would be better to have one side of the bag already open and the other attached with the perforated line. The force of the detector being introduced in to the dispensing system and pushing against the lower edge of the opened bag will provide the necessary force to tear off the rear side of the protective bag from its set of bags. The movement of the detector will be stopped when the detector edge touches the lower end of the dispensing system cavity or the receptacle [1099], preventing it from dropping onto the floor.

Since the configurations and embodiments disclosed are mounted at the outside of a cart, the storage space for the protective bags will enjoy much better temperature conditions in comparison with any possible space located inside the cart itself. The relatively open structures of the protective bag dispensing systems allow better access to cooler ambient temperatures in comparison with the internals of a mobile x-ray device cart.

## Claims

1. A protective bag dispensing system [10] for bagging a flat panel cassette detector of a mobile x-ray apparatus, comprising:
- a back panel element [102] mountable against an external and operator-accessible side of a cart [40] of said mobile x-ray apparatus, said back panel element [102] having dimensions which are exceeding the size of a protective bag [500] being dispensed,
- a front panel element [101] joined to said back panel element [102] at the bottom side of both panels forming a cavity for supporting and accommodating an X-ray detector [20] during a bagging process,
- a bag holding means [107] for hanging down a stack of multiple protective bags [500], said protective bags individually and removably attached from their top sides allowing an individual bag to be removed from the stack while inserting said X-ray detector into the first available protective bag,
- said protective bags exceeding the size of the mobile x-ray detector being bagged,
and **characterized in that**:
the back panel element comprises at least two segments arranged in a hinged configuration.

2. System according to Claim 1, wherein the back panel element comprises at least an upper [1022] and a lower [1021] segment, said upper segment comprising a fixing means [103] to attach said upper segment to an available side panel of the cart.

3. System according to Claim 2, wherein the fixing means [103] comprises a part which is removably inserted and fixed into an existing detector storage bin of the cart.

4. System according to Claim 3, in which said removable inserted fixing means is lockable into the existing detector storage bin by a mechanism which expands in at least 2 opposite directions and fixes itself against the interior walls of said storage bin.

5. System according to Claim 2, wherein said lower segment aligns with the upper segment to form a single straight segment when the dispensing system is in an open position.

6. System according to Claim 5, wherein said lower segment of said back panel element hinges towards a wall of the cart when the dispensing system is in a closed position.

7. System according to Claim 1 wherein the back panel element consists of a rigid rectangular panel element made of a flexible material such that it can flex in its entirety towards a wall of the cart.

8. System according to claim 6 comprising a magnet [108] which attracts the lower segment so that the lower segment is hinged towards the cart wall and remains in a closed position.

9. System according to any of the previous claims comprising two spacer elements [104] arranged at both sides of the back panel element separating an upper area of the front panel element from the back panel element at a distance suitable to allow insertion of the X-ray detector into the cavity.

10. System according to claim 9 wherein the front panel element and the two spacer elements are substituted with a rigid strap arranged at upper area of the back panel element.

11. System according to any of the previous Claims, wherein said back panel comprises at the bottom a rim [106] for supporting a detector cassette.

## Patentansprüche

1. Ein Schutzbeutelausgabesystem [10] zum Verpacken eines Flachschirmkassettendetektors einer mobilen Röntgenvorrichtung, umfassend:
- ein rückseitiges Plattenelement [102], das gegen eine externe und für einen Operator zugängliche Seite eines Wagens [40] der mobilen Röntgenvorrichtung montiert werden kann, wobei die Abmessungen des rückseitigen Plattenelements [102] die Größe eines ausgegebenen Schutzbeutels [500] übersteigt,
- ein vorderseitiges Plattenelement [101], das an der Bodenseite beider Platten mit dem rückseitigen Plattenelement [102] verbunden ist und eine Aussparung bildet, die einen Röntgendetektor [20] während eines Verpackungsvorgangs unterstützt und aufnimmt,
- ein Beutelhaltemittel [107], an dem ein Stapel mehrfacher Schutzbeutel [500] herunterhängt, wobei die Schutzbeutel separat und lösbar an ihren Oberseiten befestigt sind, wodurch jeweils ein einzelner Beutel vom Stapel gelöst wird, während der Röntgendetektor in den ersten verfügbaren Schutzbeutel eingeführt wird,
- wobei die Größe der Schutzbeutel die Größe des zu verpackenden mobilen Röntgendetektors übersteigt,
und **dadurch gekennzeichnet, dass**:
das rückseitige Plattenelement mindestens zwei, in einer Gelenkkonfiguration angeordnete Segmente umfasst.

2. System nach Anspruch 1, wobei das rückseitige Plattenelement mindestens ein oberes Segment [1022] und ein unteres Segment [1021] umfasst, wobei das obere Segment ein Befestigungsmittel [103], mit dem das obere Segment mit einer verfügbaren Seitenplatte des Wagens verbunden wird, umfasst.

3. System nach Anspruch 2, wobei das Befestigungsmittel [103] einen Teil, der lösbar in einen vorliegenden Detektorlagerbehälter des Wagens eingeführt und befestigt wird, umfasst.

4. System nach Anspruch 3, in dem das lösbare eingeführte Befestigungsmittel mittels eines Mechanismus, der sich in mindestens 2 entgegengesetzte Richtungen ausdehnt und sich selbst gegen die Innenwände des Lagerbehälters befestigt, in den vorliegenden Detektorlagerbehälter verriegelt werden kann.

5. System nach Anspruch 2, wobei sich das untere Segment dann mit dem oberen Segment ausrichtet und dabei ein einzelnes geradliniges Segment bildet, wenn sich das Ausgabesystem in einer offenen Position befindet.

6. System nach Anspruch 5, wobei das untere Segment des rückseitigen Plattenelements dann zu einer Wand des Wagens schwenkt, wenn sich das Ausgabesystem in einer geschlossen Position befindet.

7. System nach Anspruch 1, wobei das rückseitige Plattenelement aus einem steifen rechteckigen Plattenelement aus einem biegsamen Material aufgebaut ist, wodurch es sich in seiner Gesamtheit zu einer Wand des Wagens biegen kann.

8. System nach Anspruch 6, umfassend einen Magneten [108], der das untere Segment derart anzieht, dass das untere Segment zur Wagenwand schwenkt und in einer geschlossen Position verbleibt.

9. System nach einem der vorstehenden Ansprüche, umfassend zwei Abstandshalterelemente [104], die an beiden Seiten des rückseitigen Plattenelements angeordnet sind und einen oberen Bereich des vorderseitigen Plattenelements vom rückseitigen Plattenelement an einem geeigneten Abstand trennen, damit der Röntgendetektor in die Aussparung eingeführt werden kann.

10. System nach Anspruch 9, wobei das vorderseitige Plattenelement und die zwei Abstandshalterelemente durch ein steifes, am oberen Bereich des rückseitigen Plattenelements angeordnetes Band ersetzt werden.

11. System nach einem der vorstehenden Ansprüche, wobei die rückseitige Platte am Boden einen Rand [106], der eine Detektorkassette trägt, umfasst.

## Revendications

1. Système de distribution de sacs de protection [10] pour ensacher un capteur plan à cassette d'un appareil à rayons X mobile, comprenant :
- un élément de panneau arrière [102] pouvant être monté contre un côté externe et accessible à un opérateur d'un chariot [40] de l'appareil à rayons X mobile, les dimensions dudit élément de panneau arrière [102] dépassant la taille d'un sac de protection [500] à distribuer,
- un élément de panneau avant [101] raccordé à l'élément de panneau arrière [102] du côté inférieur des deux panneaux, formant une cavité supportant et recevant un capteur de rayons X [20] lors d'un processus d'ensachage,
- un moyen de retenue de sac [107] duquel pend une pile d'une multitude de sacs de protection [500], lesdits sacs de protection étant fixés de manière individuelle et amovible par leurs côtés supérieurs, permettant ainsi de reprendre chaque fois un seul sac de ladite pile pendant que le capteur de rayons X est inséré dans le premier sac de protection disponible,
- la taille des sacs de protection dépassant la taille du capteur de rayons X mobile à ensacher,
et **caractérisé en ce que**:
ledit élément de panneau arrière comprend au moins deux segments disposés dans une configuration articulée.

2. Système selon la revendication 1, **caractérisé en ce que** ledit élément de panneau arrière comprend au moins un segment supérieur [1022] et un segment inférieur [1021], ledit segment supérieur comprend un moyen de fixation [103] servant à raccorder le segment supérieur à un panneau latéral disponible du chariot.

3. Système selon la revendication 2, **caractérisé en ce que** ledit moyen de fixation [103] comprend une partie pouvant être insérée et fixée de manière amovible dans un réceptacle de stockage de capteur présent du chariot.

4. Système selon la revendication 3, dans lequel ledit moyen de fixation inséré amovible peut être verrouillé dans le réceptacle de stockage de capteur présent au moyen d'un mécanisme qui se dilate dans au moins 2 directions opposées et qui se fixe lui-même contre les parois intérieures du réceptacle de stockage.

5. Système selon la revendication 2, **caractérisé en ce que** le segment inférieur s'aligne avec le segment supérieur et forme ainsi un seul segment rectiligne lorsque le système de distribution se trouve en position ouverte.

6. Système selon la revendication 5, **caractérisé en ce que** le segment inférieur de l'élément de panneau arrière s'articule vers une paroi du chariot lorsque le système de distribution se trouve en position fermée.

7. Système selon la revendication 1, **caractérisé en ce que** l'élément de panneau arrière est composé d'un élément de panneau rectangulaire rigide réalisé en un matériau flexible permettant ainsi de se plier dans son entièreté vers une paroi du chariot.

8. Système selon la revendication 6 comprenant un aimant [108] attirant le segment inférieur de façon à ce que le segment inférieur s'articule vers la paroi de chariot et se maintient en position fermée.

9. Système selon l'une quelconque des revendications précédentes, comprenant deux éléments écarteurs [104] disposés des deux côtés de l'élément de panneau arrière et séparant une zone supérieure de l'élément de panneau avant de l'élément de panneau arrière à une distance appropriée pour que le capteur de rayons X puisse être inséré dans la cavité.

10. Système selon la revendication 9, **caractérisé en ce que** l'élément de panneau avant et lesdits deux éléments écarteurs sont substitués par une bande rigide disposée de la zone supérieure de l'élément de panneau arrière.

11. Système selon l'une quelconque des revendications précédentes, **caractérisé en ce que** le panneau arrière comprend sur son côté inférieur un bord [106] servant à supporter une cassette de capteur.
